# EUROPEAN PATENT APPLICATION

(11) **EP 4 218 930 A1**
(43) Date of publication of application: **02.08.2023**
(21) Application number: 22382078.8
(22) Date of filing: 31.01.2022
(51) Int. Cl.: A61P 35/00, C07K 16/28, C07K 16/40

(54) **TREATMENT AND PROGNOSIS OF CANCER**

(71) Applicant: Cancerappy, S.L., 48950 Erandio (Bizkaia) (ES)
(72) Inventor: The designation of the inventor has not yet been filed
(74) Representative: Herrero & Asociados, S.L.

(57) **Abstract**

The present invention refers to the field of cancer treatment and prognosis. In particular, it refers to TMPRSS6 and/or CLDN10 as prognostic markers and as selective targets for the vectorization of therapeutic agents towards cancer cells.

## Description

### FIELD OF THE INVENTION

The present invention refers to the field of cancer treatment and prognosis. In particular, it refers to the use of TMPRSS6 and/or CLDN10 as prognostic markers and as selective targets for the vectorization of therapeutic agents towards cancer cells.

### BACKGROUND OF THE INVENTION

Identification of the key molecular alterations that drive oncogenesis is one of the main objectives in current cancer research. These alterations are essential to regulate tumour generation and progression but, at the same time, generate vulnerabilities that can be therapeutically exploited. Thus, in the last few decades much effort has been put on the development of anticancer treatments selectively targeting gene products that become dysregulated as a consequence of those oncogenic alterations. This strategy, combined with the generalization of the use of molecular diagnosis, has led to the application of more individualized and selective treatments which has conducted to a very significantly improvement in patient survival in several types of cancer.

Recently, a novel example of this type of selective therapies is the development of agents against the Kirsten Rat Sarcoma 2 Viral Oncogene Homolog (K-RAS, or KRAS hereinafter) pG12C mutation in non-small cell lung cancer (NSCLC). K-RAS is a small G protein, and one of the most frequently mutated genes in cancer. Most mutation on this gene occur at the Glycine 12 (G12) residue, which renders K-RAS constitutively active by favouring its GTP-bound active form. Activated GTP-bound K-RAS stimulates many different intracellular signalling pathways such as the RAF1/MEK/ERK and PI3K cascades that are directly involved in the regulation of cell proliferation, motility, etc. The paramount importance of K-RAS mutations in cancer has led to a prolonged effort to develop strategies to target this protein, which however was considered to be undruggable due to its structural characteristics.

Interestingly, the presence of a thiol group at Cys 12 of K-RAS G12C offered an opportunity to develop inhibitors against this specific mutant, which is present with a notable frequency in certain cancer types and specifically in NSCLC. Following this strategy, a new family of compounds aimed at targeting K-RAS G12C has been developed. One of these compounds (AMG-510 or Sotorasib) acts by holding K-RAS G12C in its GDP-bound inactive form has recently been granted accelerated approval for the treatment of K-RAS G12C mutated locally advanced or metastatic NSCLC using a specific companion diagnostic. Likewise, other compounds acting on the same target are currently in clinical development.

Targeting oncogenic membrane kinase proteins with antibodies or small molecule signalling mediators binding to the kinase pocket has shown to induce clinical benefit. However, this effect is short lived, and resistance frequently appears within a short period of time. Several mechanisms of resistance have been described, and most overcome the activity inhibited by the compound through the activation of alternative pathways, acquisition of secondary mutations at the kinase pocket, or the presence of gene amplifications or alternative splicing, among others. Among these, the development of secondary mutations is a key mechanism described for inhibitors against K-RAS G12C. In this context, the identification of smart combinations - including the association with novel immunotherapies - is a main goal in this particular clinical scenario.

An interesting approach to overcome resistance due to specific mutations is reducing the abundance of the mutated protein by promoting its selective degradation. One strategy to follow this approach, and that is currently intensively investigated, is the utilization of Proteolysis Targeting Chimeras (PROTAC) compounds which through their binding with specific E3 ligases, induce the ubiquitination and subsequent degradation of the target protein.

PROTACs have been recently synthetized with this aim of overcoming resistance against K-RAS G12C inhibitors (Zeng M, et al. 2020). However, these compounds, like many others agents against pan-essential genes, suffer from potential toxicity, as they act against non-transformed tissues with a potent off-target effect, limiting their clinical development (Settleman J, et al. 2021). One of the ways to avoid this problem is the identification of proteins that are exclusively present in the tumour surface and that could therefore be used as selective targets for the vectorization of novel compounds. In this context, an important requisite is the identification of proteins specifically expressed at the membrane of tumour cells.

Taken together, there is a need for new medical treatments for cancer, in particular for those ones characterized by having a mutation at position G12 in *KRAS* gene which have a poor prognosis.

### SUMMARY OF THE INVENTION

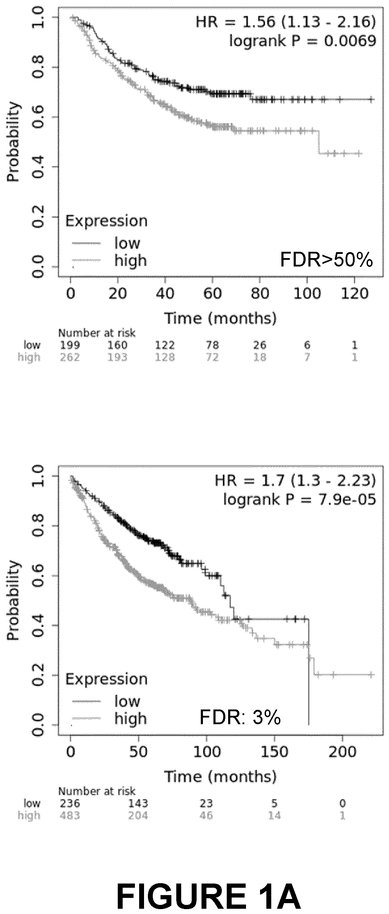

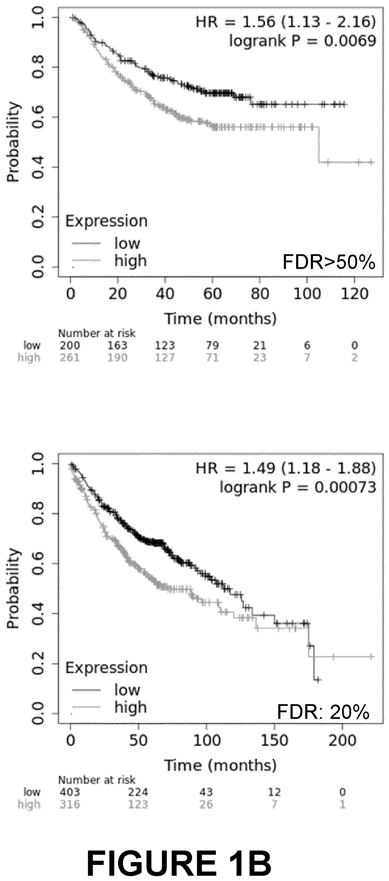

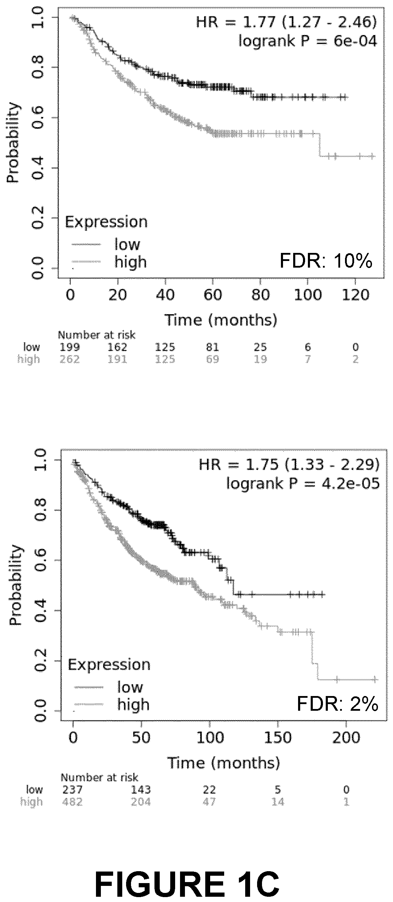

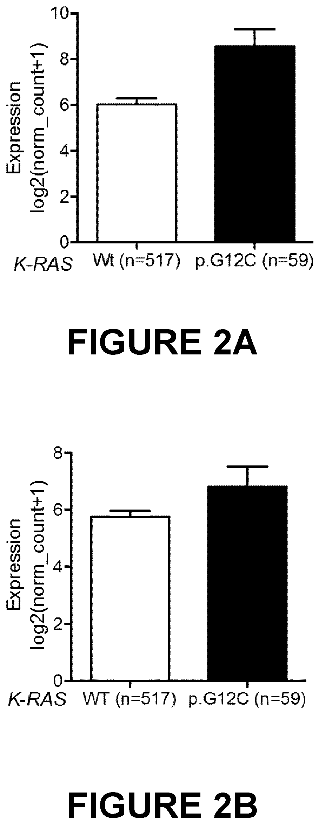

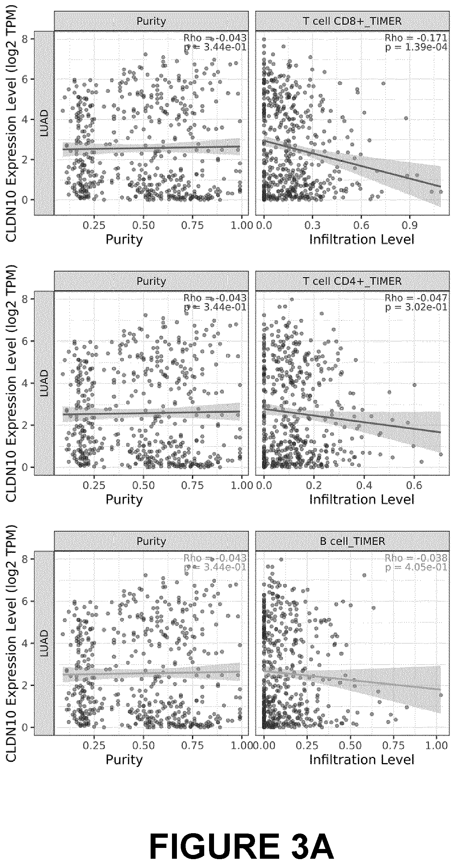

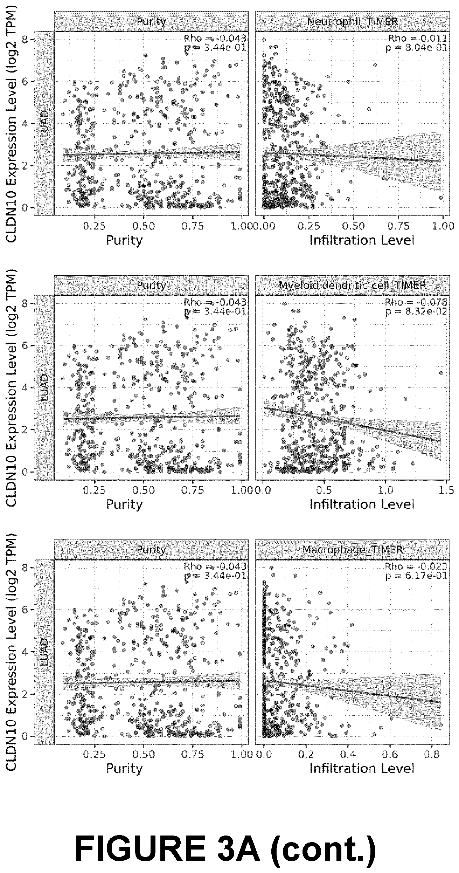

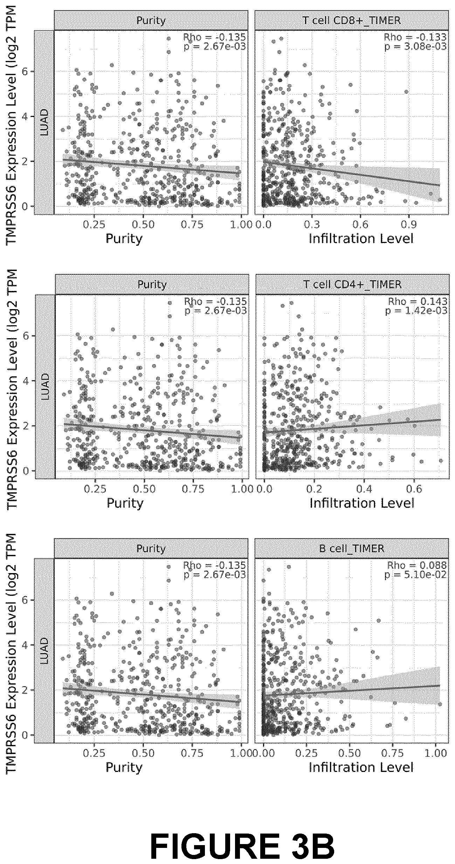

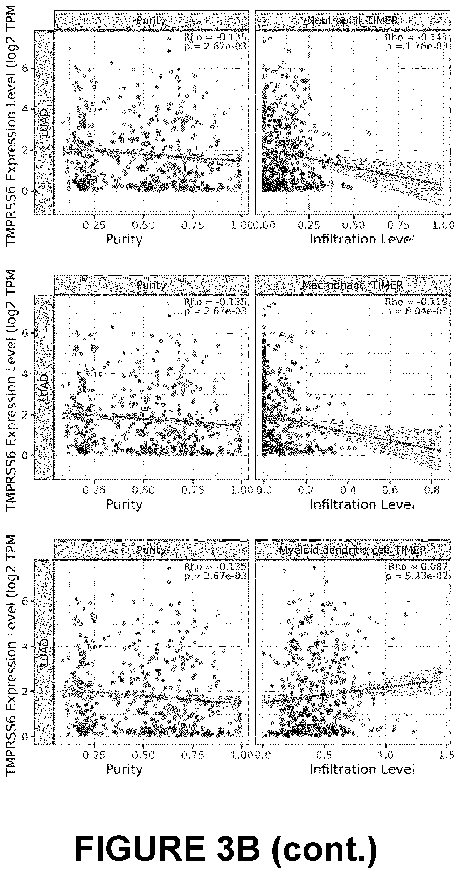

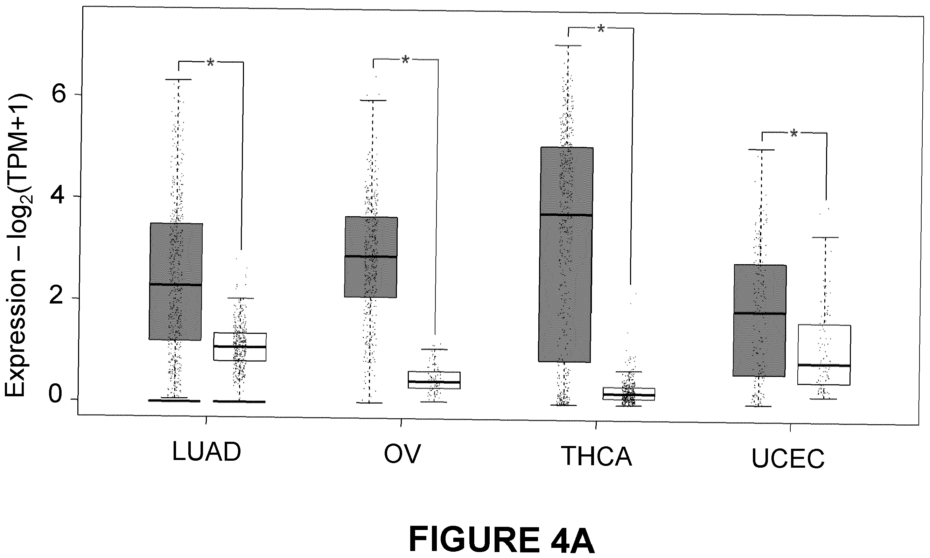

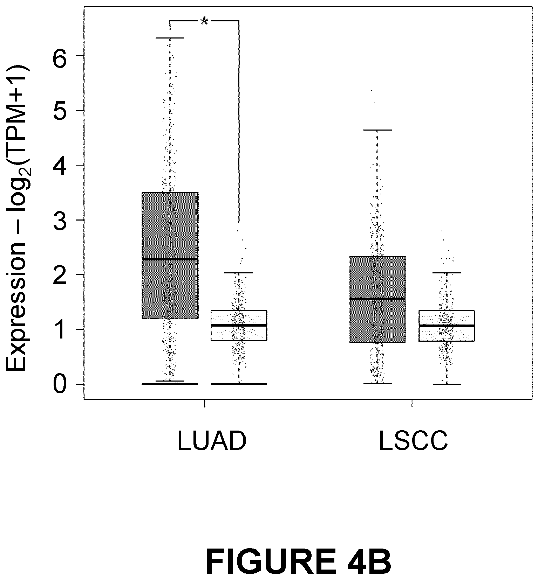

The selection of a therapeutic treatment for each individual case of cancer depends on clinical and pathological variables which are not always able to predict the therapeutic efficacy of a treatment or to establish a prognosis concerning the progress of a patient suffering that type of cancer. The inventors have surprisingly found that an increase of the expression level of the TMPRSS6 and/or CLDN10 gene products in specific cancers, particularly in LUAD, is associated with poor prognosis (Figure 1). Moreover, TMPRSS6 and/or *CLDN10* genes are highly expressed in LUAD tumours harboring the mutation KRAS G12C in comparison with wild type KRAS LUAD ones (Figure 2), and TMPRSS6 and CLDN10 proteins are selectively expressed at the cellular membrane in LUAD tumours. Interestingly, TMPRSS6 and/or *CLDN10* gene expression is not associated with any immune population suggesting that they are present in the primary tumour (Figure 3). Finally, *TMPRSS6* and/or *CLDN10* genes are also highly and selectively expressed in other tumours (Figure 4). Thus, TMPRSS6 and/or CLDN10 gene products are useful as selective targets for the delivery of therapeutic agents to said tumour cells, and, therefore, molecules with affinity and/or specifically binding to said TMPRSS6 and/or CLDN10 gene products are useful in the treatment of said cancers.

A **first aspect** the present invention refers to an antibody for use in the treatment of cancer, wherein the antibody is an anti-TMPRSS6, and/or an anti-CLDN10, and the cancer is selected from lung adenocarcinoma (LUAD), ovarian serous cystadenocarcinoma (OV), thyroid carcinoma (THCA) and uterine corpus endometrial carcinoma (UCEC).

A **second aspect** of the present invention refers to functional nucleic acid for use in the treatment of cancer, characterized in that a transcript of TMPRSS6 and/or CLDN10 is the target of said functional nucleic acid, preferably the functional nucleic acid is selected from an siRNA, shRNA or miRNA, and the cancer is selected from LUAD, OV, THCA and UCEC.

A **third aspect** of the present invention refers to a pharmaceutical composition for use in the treatment of cancer, wherein the pharmaceutical composition comprises the antibody defined in the first aspect of the invention and/or the functional nucleic acid defined in the second aspect of the invention, and a pharmaceutically acceptable carrier, and the cancer is selected from LUAD, OV, THCA and UCEC.

A **fourth aspect** of the present invention refers to a prognostic method for the prognosis of LUAD comprising the steps of:
a. quantifying the expression level of TMPRSS6 and/or CLDN10, in a sample isolated from an individual who has LUAD (test sample);
b. comparing the levels obtained in step (a) with a reference value or with the expression levels of TMPRSS6 and/or CLDN10 in a reference sample; and
c. assigning the individuals having an expression level of TMPRSS6 and/or CLDN10 obtained in step (b) that is greater than the reference value or the expression level of the reference sample to the group of individuals with a poor prognosis.

A **fifth aspect** of the invention refers to TMPRSS6 and/or CLDN10 for use in the treatment of cancer as target molecule(s) for delivery of a therapeutic agent or therapy, wherein the cancer is selected from LUAD, OV, THCA and UCEC.

Other objects, features, advantages and aspects of the present application will become apparent to those skilled in the art from the following description and appended claims.

### BRIEF DISCUSSION OF THE FIGURES

**Figure 1** depicts Kaplan-Meier survival plots showing the association between the single gene (*CLDN10* in **A**; and *TMPRSS6* in **B**) or combined gene expression levels (*CLDN10* and *TMPRSS6* in **C**) with First progression (FP, above panel, n=461) and Overall Survival (OS, below panel, n=719) for lung adenocarcinoma (LUAD). Low expression levels in black line and high expression levels in grey line.
**Figure 2** depicts bar graphs showing the expression of *CLDN10* gene (in **A**), and *TMPRSS6* gene (in **B**) in LUAD tumour samples comparing those that harbor (black) or not (white) the G12C mutation in *KRAS* gene. Error bars represent mean +/- 95% confidence Interval.
**Figure 3** depicts dot plots with regression line showing no correlation between *CLDN10* (**A**) and *TMPRSS6* (**B**) gene expression with infiltration levels of most immune populations in LUAD: T cell CD8+, T cell CD4+, B cell, Neutrophil, Myeloid dendritic cell, and Macrophage (from top to bottom).
**Figure 4** depicts bar graphs showing the expression of *CLDN10* gene and *TMPRSS6* gene combined in those cancer types where expression is higher in tumour tissue (T) samples (grey) than in normal (N) tissue samples (white). (**A**) The cancers analysed are LUAD (n(T)=483; n(N)=347), OV (n(T)=426; n(N)=88), THCA (n(T)=512; n(N)=337), and UCEC (n(T)=174; n(N)=91). (**B**) The cancers analysed are LUAD (n(T)=483; n(N)=347) and lung squamous cell carcinoma (LUSC, (n(T)=486, n(N)=338). (*) means statistically significant.

### DETAILED DESCRIPTION OF THE INVENTION

Unless defined otherwise, all the technical and scientific terms used herein have the same meaning as commonly understood by the skilled in the art to which this invention belongs. To facilitate understanding and clarify the meaning of specific terms in the context of the present invention, the following definitions and particular and preferred embodiments thereof, applicable to all the embodiments of the different aspects of the present invention, are provided:
The singular forms "a", "an" and "the" include their corresponding plurals unless the context clearly dictates otherwise.

The term "and/or" means any one of the items, any combination of the items, or all of the items with which this term is associated.

The term "TMPRSS6" is intended here to mean the gene encoding the Type II transmembrane serine protease 6 (TMPRSS6) protein, also known as matriptase-2; transmembrane protease serine 6; membrane-bound mosaic serine proteinase matriptase-2; and MT2, and the protein. TMPRSS6 is involved in matrix remodelling and it has an essential role in iron homeostasis. It has been linked to iron-refractory iron deficiency anaemia. However, its role in cancer is less understood, and there is very limited published data. The human TMPRSS6 gene sequence is typically referenced under the gene number ID 164656 (NCBI). The human TMPRSS6 protein sequence is typically referenced under the UniProt number Q8IU80.

The term "CLDN10" is intended here to mean the gene encoding claudin 10 protein and the protein. CLDN10 belongs to the claudin family which are integral membrane proteins and components of tight junction strands. They play and important role in cell polarity and signal transduction, and they are present in the intercellular space between epithelial or endothelial components. There are different reports that establish a correlation between the expression of CLDN10 and survival, with some articles associating their increased levels with favourable prognosis (Xiang Z et al. 2020, Yang W, et al. 2021), and others with detrimental outcome (Zhou Y, et al. 2018). The human CLDN10 gene sequence is typically referenced under the gene number ID 9071 (NCBI). The human CLDN10 protein sequence is typically referenced under the UniProt number P78369. The terms "CLDN10 " and "TMPRSS6" as used herein not only include the human gene and protein but also their orthologues from other species such as dogs, mice, rats, etc., as well as functionally equivalent variants thereof. Preferably they refer to the human gene and protein. In a particular embodiment of the invention, the amino acid sequence of TMPRSS6 protein comprises or consists of sequence SEQ ID NO: 1, or a functionally equivalent variant thereof. In another particular embodiment of the invention, the amino acid sequence of CLDN10 protein comprises or consists of sequence SEQ ID NO: 2, or a functionally equivalent variant thereof.

As it is used herein, the term "protein" relates to a molecular chain of amino acids, bound by covalent or noncovalent bonds. The term furthermore includes all the forms of physiologically relevant post-translational chemical modifications, for example, glycosylation, phosphorylation or acetylation, etc., provided that the functionality of the protein is maintained.

"Functionally equivalent variant of a protein" is understood as a protein the amino acid sequence of which (i) is substantially homologous to the amino acid sequence of the TMPRSS6 or CLDN10 protein and (ii) performs the same functions as the TMPRSS6 or CLDN10 protein.

An amino acid sequence is substantially homologous to a determined amino acid sequence when it presents a degree of identify of at least 70%, at least 75%, at least 80%, preferably of at least 85%, more preferably of at least 90%, still more preferably of at least 95%, 97%, 98% or 99%, with respect to said determined amino acid sequence. The degree of identity between two amino acid sequences can be determined by conventional methods, for example, by means of standard sequence alignment algorithms known in the state of the art, such as, for example, BLAST (Altschul S.F. et al. Basic local alignment search tool. J Mol Biol. 1990 Oct 5; 215(3):403-101).

An "effective amount" refers to an amount effective to treat a disease, disorder, and/or condition, or to bring about a recited effect, such as activation or inhibition. For example, an effective amount can be an amount effective to reduce the progression or severity of the condition or symptoms being treated. Determination of a therapeutically effective amount is well within the capacity of the skilled in the art.

In one embodiment, an effective amount refers to an amount of the active agent described herein that are effective, either alone or in combination with a pharmaceutical carrier, upon single- or multiple-dose administration to a cell or a subject, e.g., a patient, at inhibiting the growth or proliferation, inducing the killing, or halting the growth of hyperproliferative cells. Such growth inhibition or killing can be reflected as a prolongation of the survival of the subject, or any improvement in the prognosis of the subject relative to the absence of such treatment.

The terms "treating", "treat" and "treatment" include (i) inhibiting the disease, pathologic or medical condition or arresting its development; (ii) relieving the disease, pathologic or medical condition; and/or (iii) diminishing symptoms associated with the disease, pathologic or medical condition. Thus, the terms "treat", "treatment", and "treating" can include lowering, stopping or reversing the progression or severity of the condition or symptoms being treated. In some embodiments, the terms "treatment", "treat" or "treated" can refer to (i) a reduction or elimination of symptoms or the disease of interest (therapy) or (ii) the elimination or destruction of the tumour (cure).

The terms "inhibit", "inhibiting", and "inhibition" refer to the slowing, halting, or reversing the growth or progression of a disease, condition, or group of cells. The inhibition can be greater than about 20%, 40%, 60%, 80%, 90%, 95%, or 99%, for example, compared to the growth or progression that occurs in the absence of the treatment. Additionally, the terms "induce," "inhibit," "potentiate," "elevate," "increase," "decrease," or the like denote quantitative differences between two states, and can refer to at least statistically significant differences between the two states. For example, "an amount effective to inhibit the growth of hyperproliferative cells" means that the rate of growth of the cells can be, in some embodiments, at least statistically significantly different from the untreated cells. Such terms can be applied herein to, for example, rates of proliferation. The phrase "inhibiting the growth or proliferation" of the hyperproliferative cell, e.g. neoplastic cell, refers to the slowing, interrupting, arresting, or stopping its growth and metastasis, and does not necessarily indicate a total elimination of the neoplastic growth. The term "antibody" refers particularly to an immunoglobulin. As used herein, the term "immunoglobulin" is used consistent with its well-known meaning in the art and comprises two heavy chains and two light chains. Methods for making antibodies are well-known by the skilled in the art. Preferably, the antibody is a monoclonal antibody and/or it is a human antibody, chimeric antibody and/or humanized antibody.

By "chimeric antibody" is meant an antibody that is composed of variable regions from a murine immunoglobulin and of constant regions of a human immunoglobulin. This alteration consists simply of substituting the constant region of a human antibody for the murine constant region, thus resulting in a human/murine chimera which may have sufficiently low immunogenicity to be acceptable for pharmaceutical use.

By "humanized antibody" is meant an antibody that is composed partially or fully of amino acid sequences derived from a human antibody germline by altering the sequence of an antibody having non-human complementarity determining regions (CDR). This humanization of the variable region of the antibody and eventually the CDR is made by techniques that are by now well known in the art.

The term "functional fragment" as used herein refers to an antibody fragment capable of recognizing the protein of interest, particularly to an antigen-binding fragment. Such fragments can be simply identified or produced by the skilled person and comprise, as an example, Fab fragment (that can be produced by papain digestion), Fab ' fragment (that can be produced by pepsin digestion and partial reduction), F(ab ')2 fragment (that can be produced by pepsin digestion), Facb (that can be produced by plasmin digestion), Fd (that can be produced by pepsin digestion, partial reduction and reaggregation), scFv (single chain Fv, produced by molecular biology techniques) fragment, diabodies, monobodies, and nanobodies. Preferably the functional fragment is selected from the group comprising or consisting of Fab, Fab', F(ab')2, Facb, Fd, scFv, diabodies, monobodies including VHH fragments and human VH fragments, and nanobodies. The term "diabodies" refers to small antibody fragments with two antigen-binding sites, which fragments comprise a heavy-chain variable domain (VH) connected to a lightchain variable domain (VL) in the same polypeptide chain (VH - VL). Preferably, by using a linker that is too short to allow pairing between the two domains on the same chain, the domains are forced to pair with the complementary domains of another chain and create two antigen-binding sites.

The term "monobodies" as used herein, refers to antigen binding molecules with a heavy chain variable domain and no light chain variable domain. A monobody can bind to an antigen in the absence of light chain and typically has three CDR regions designated CDRH1, CDRH2 and CDRH3. Monobodies include "camelid monobodies" such as VHH fragments obtained from a source animal of the camelid family, including animals with feet with two toes and leathery soles. Animals in the camelid family include camels, llamas, and alpacas. It has been reported that camels *(Camelus dromedaries* and *Camelus bactrianus)* often lack variable light chain domains when material from their serum is analysed, suggesting that sufficient antibody specificity and affinity can be derived from VH domains (three CDR loops) alone. Monobodies also include modified VH from various animal sources, in particular mammals (for example mouse, rat, rabbit, horse, donkey, bovine or human), which can bind to an antigen in the absence of VL. Preferably, the VH is modified in positions at the VL interface to provide for binding of the VH to antigen in absence of the VL. One skilled in the art is able to optimize a human VH by substitution of some important residues (by "camelization") to mimic camelid antibody heavy chains naturally devoid of light chain partners. This permits to obtain antibody functional fragments with stability properties and expression levels similar to camelid VHH while keeping the recognition properties of the antibody fragment, including high affinity and high specificity and decreasing immunogenicity.

The term "nanobody" refers to heavy chain antibodies (HCAB) based on distinct immunoglobulin structures found naturally in sharks and camelid. The structure of HCAB is unique, composed of a single heavy chain with one variable domain (VHH). The VHH is equivalent to the Fab fragment of IgG antibodies. Nanobodies are smaller and have longer VHH domains facilitating their reach to targets inaccessible to conventional antibodies.

The term "prognosis" as used herein refers to predicting disease progression or outcome. More specifically, "prognostic markers" may refer to patient characteristics that predict outcome (usually survival) independent of the treatment. The goal of identifying prognostic markers is to define patient subpopulations with significantly different anticipated outcomes, which might benefit from different therapies. Good prognostic patients may not require additional treatment, while poor prognostic patients may derive improved survival benefit from closer clinical follow up or a personalized therapeutic strategy. Poor prognosis is defined by high rate of relapse or death of the patient suffering from said cancer and/or low overall survival.

The term *"in vitro",* as used herein, refers to the fact that a method is not carried out on the body of a human subject, but on samples isolated from said subject, and already present in a laboratory tool, such as a test tube, dish, slide or plate.

The term "expression level", as used herein, refers to the expression level of a gene product, more specifically to a measurable quantity of a gene product produced by the gene in a sample of the subject, wherein the gene product can be a transcriptional product or a translational product. As understood by the skilled in the art, the gene expression level can be quantified by measuring the RNA, particularly messenger RNA (mRNA), levels of said gene, or of the protein encoded by said gene. In a particular embodiment, the expression level of said gene is determined by measuring the mRNA levels of said gene or the levels of the protein encoded by said gene.

The expression "reference value" or "reference level" of the expression level of a gene product refers to the expression level of a gene product in one or more samples used as reference (and referred to as "reference sample" or "control sample") used to evaluate the expression level of a gene product in a sample of interest (referred to as "test sample"). As understood by the skilled in the art, the reference and test samples should derive from the same type of sample (e.g., body fluid or tissue).

The reference value or reference level can be an absolute value; a relative value; a value that has an upper or a lower limit; a range of values; an average value; a median value; a mean value; or a value as compared to a particular control or baseline value. A reference value can be based on an individual sample value or on a large number of samples, such as from population of subjects of the chronological age matched group, or based on a pool of samples.

Preferably, the "reference sample" is a sample obtained from a subject not having the disease/condition. As the skilled in the art will understand, the reference expression level can also consist on the mean of expression levels determined in a group of reference samples as just defined. In fact, it is preferred to use the mean of a group of reference samples to reduce the variability due to the difference in sample donors (age, sex, etc.) and difference in the quality of the sample obtained from the different donors.

The term "biological sample" or "sample" refers to biological material isolated from a subject. The biological sample contains any biological material suitable for detecting TMPRSS6 and/or CLDN10. Samples include, but are not limited to, primary or cultured cells or cell lines, cell supernatants, cell lysates, serum, plasma, vitreous fluid, lymph fluid, synovial fluid, follicular fluid, seminal fluid, amniotic fluid, milk, whole blood, blood plasma, blood serum, blood-derived cells, urine, cerebro-spinal fluid, saliva, sputum, tears, perspiration, mucus, tumour lysates, tissue culture medium, tissue extracts such as homogenized tissue, tumour tissue, cellular extracts, and combinations thereof. Preferably, the sample is whole blood, a bronchial aspirate (BAS), a bronchioalveolar lavage (BAL), or a tumour tissue. The sample is preferably a fresh sample, a frozen fresh sample or a sample embedded in paraffin. Preferably, the sample is pre-treated with RNA later for better preservation of the RNA.

According to the prognostic method of the invention, the level or the expression level of TMPRSS6 and/or CLDN10 is considered "greater" or "increased" when the expression level of TMPRSS6 and/or CLDN10 in a sample is higher than its reference value, preferably with an increase statistically significant. In a particular embodiment, the expression level is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value. Preferably, the expression level is considered to be higher than its reference value when it is at least 1.5 times, 2.0 times or 2.5 times higher than its reference value and, more preferably, the increase is statistically significant.

The inventors have identified TMPRSS6 and CLDN10 gene products as prognosis markers, in particular an increase in the expression of any of these gene products or their combination is indicative of bad or poor prognosis (Figure 1). Moreover, the inventors have surprisingly identified TMPRSS6 and CLDN10 as novel genes upregulated in various cancers (Figure 4), and they are specifically overexpressed in LUAD harboring the KRAS G12C mutation in comparison with wild type KRAS LUAD (Figure 2). Interestingly, the proteins encoded by said genes are membrane proteins that can be used as targets for the design of antibody guided compounds, or as surrogates of immune activation, to select patients or explore combinations with novel immune therapies. Advantageously, the overexpression of neither CLDN10 nor TMPRSS6 are associated with any immune population, suggesting that they are present in the primary tumour and not in the stromal compartment, thus, allowing to target the tumour core specifically (Figure 3).

Thus, in a **first aspect**, the present invention refers to an antibody for use in the treatment of cancer, wherein the antibody is an anti-TMPRSS6, and/or an anti-CLDN10, and the cancer is selected from LUAD, OV, THCA and UCEC.

About 80% to 85% of lung cancers are NSCLC. The main subtypes of NSCLC are LUAD, LUSC, and lung large cell carcinoma (LLCC), being LUAD the most abundant subtype (approximately 60%). These subtypes, which start from different types of lung cells are grouped together as NSCLC because their treatment and prognoses are often similar. LUADs start in the cells that would normally secrete substances such as mucus. LUSCs start in squamous cells, which are flat cells that line the inside of the airways in the lungs. LLCCs can appear in any part of the lung. It tends to grow and spread quickly, which can make it harder to treat. A subtype of large cell carcinoma, known as large cell neuroendocrine carcinoma, is a fast-growing cancer that is very similar to small cell lung cancer.

Around 35% of LUAD patient have mutations at K-RAS (hereinafter referred as LUAD KRAS). The inventors have seen that the most frequent mutation is the one at position 12 that changes Glycine for different amino acids (hereinafter referred to as LUAD KRAS G12X, wherein X is any amino acid except Glycine), such as G12C observed in 44.44% of patients, followed by G12V in 19.44%, G12D in 9.26% and G12A in 8.80 % of patients. In the same position, there are also other mutations less frequently observed: G12S (2.31%), G12F (0.93%), G12R (0.46%), G12Y (0.46%). Thus, in a particular embodiment of the first aspect of the invention the LUAD is LUAD KRAS G12X, more particularly LUAD KRAS G12C, LUAD KRAS G12V, LUAD KRAS G12D or LUAD KRAS G12A (i.e. X is selected from the group consisting of C, V, D or A, respectively).

In a preferred embodiment of the first aspect of the invention, the cancer is LUAD KRAS G12C. This cancer is, unfortunately, associated with a poor prognosis and efficient treatments remain unknown. As shown in Example 2, there is a higher expression of *CLDN10* gene (Figure 2A) and *TMPRSS6* gene (Figure 2B) when K-RAS harbors the mutation G12C in LUAD tumour samples, in comparison to wild type KRAS LUAD tumour samples.

In another preferred embodiment according to any one of the embodiments of the first aspect of the invention, the antibody is an anti-TMPRSS6 and an anti-CLDN10, i.e. a combination of both, an anti-TMPRSS6 antibody and an anti-CLDN10 antibody.

In a preferred embodiment according to any of the embodiment of the first aspect of the invention, the anti-TMPRSS6 and/or anti-CLDN10 antibodies bind to the native TMPRSS6 and/or CLDN10 protein, respectively, and/or to the extracellular part of the protein.

Anti-TMPRSS6 and anti-CLDN10 antibodies that bind to TMPRSS6 and CLDN10, respectively, are described in the state of the art and are commercially available or can be produced by the skilled in the art.

In a particular embodiment according to any one of the embodiments of the first aspect of the invention the antibody is a functional fragment of the antibody, preferably said fragment is an antigen-binding fragment, more preferably it is selected from the group comprising or consisting of Fab, Fab', F(ab')2, Facb, Fd, scFv, diabodies, monobodies including VHH fragments and human VH fragments, and nanobodies.

The antibody for use according to any of the embodiment of the fist aspect of the invention is selected from the group consisting of a monoclonal antibody, a chimeric antibody, a human antibody or a humanized antibody, preferably it is a monoclonal antibody and more preferably a monoclonal humanized antibody.

In a particular embodiment of the first aspect of the invention, the antibody is a multispecific antibody, particularly a bispecific antibody. Multispecific antibodies are monoclonal antibodies that have binding specificities for at least two different sites. Bispecific antibodies are monoclonal antibodies that have binding specificities for two different sites. In a particular embodiment, one of those two different sites is TMPRSS6 or CLDN10, acting as cancer antigen expressed by the cancer cells.

In another particular embodiment of the first aspect of the invention, the antibody is a T-cell engager. By "T-cell engager or bi-specific T-cell engager" is meant an antibody that in one site by using a specific epitope engages an immunologic cell such as a T cell, natural killer cell, T regulatory cell or macrophage, among others, and in the other site engages a tumour antigen epitope, in this case TMPRSS6 or CLDN10. With this strategy, immune effector cells are attracted to specific tumoral cells that overexpressed said proteins in the membrane.

In another particular embodiment of the first aspect of the invention, the antibody is an immunoconjugate. By "immunoconjugate" is meant an antibody conjugated to one or more heterologous molecule(s), including but not limited to a cytotoxic agent.

In another particular embodiment of the first aspect of the invention, the antibody is an antibody drug conjugate (ADC). An ADC meant an antibody that is attached to a payload, either a chemotherapy or a targeted agent, with the use of a linker that binds both structures. The payload could include a targeted agents or a chemical entity such as a PROTAC compound.

In another particular embodiment of the first aspect of the invention, the genomic structure of the receptor is used to create a chimeric antigen receptor (CAR). More particularly, the CAR is selected from CAR cell T (CAR-T), CAR macrophage (CAR-M), CAR natural killer (CAR-NK), and CAR Cytokine-induced Killer (CAR-CIK).

The first aspect of the invention also refers to a method of treating cancer in a subject in need thereof, comprising administering to the subject a therapeutically effective amount of an antibody as defined above in any of the embodiments of the first aspect of the invention, wherein the cancer is selected from LUAD, OV, THCA, and UCEC.

The first aspect of the invention also refers to the use of an antibody as defined above in any of the embodiments of the first aspect of the invention for the preparation of a medicament for the treatment cancer, wherein the cancer is selected from LUAD, OV, THCA, and UCEC.

In a particular embodiment of the first aspect according to the previous two paragraphs LUAD is LUAD KRAS G12X, more particularly LUAD KRAS G12C, LUAD KRAS G12V, LUAD KRAS G12D or LUAD KRAS G12A. Preferably, the cancer is LUAD KRAS G12C.

A **second aspect** of the present invention refers to a functional nucleic acid for use in the treatment of cancer, characterized in that a transcript of TMPRSS6 and/or CLDN10 is the target of said functional nucleic acid, and the cancer is selected from LUAD, OV, THCA, and UCEC.

In another preferred embodiment according to any of the embodiments of the second aspect of the invention, the functional nucleic acid is selected from an siRNA, shRNA or miRNA, and more preferably it is siRNA.

The second aspect of the invention also refers to a method of treating cancer in a subject in need thereof, comprising administering to the subject a therapeutically effective amount of a functional nucleic acid as defined above in any of the embodiments of the second aspect of the invention, wherein the cancer is selected from LUAD, OV, THCA, and UCEC.

The second aspect of the invention also refers to the use of a functional nucleic acid as defined above in any of the embodiments of the second aspect of the invention for the preparation of a medicament for the treatment cancer, wherein the cancer is selected from LUAD, OV, THCA, and UCEC.

In a particular embodiment of the second aspect of the invention the LUAD is LUAD KRAS G12X, more particularly LUAD KRAS G12C, LUAD KRAS G12V, LUAD KRAS G12D or LUAD KRAS G12A.

In a preferred embodiment of the first aspect of the invention, the cancer is LUAD KRAS G12C.

The antibody and functional nucleic acid defined in the first and a second aspects of the invention, respectively, can be formulated within a pharmaceutical composition. Thus, a **third aspect** of the present invention refers to a pharmaceutical composition for use in the treatment of cancer, wherein the pharmaceutical composition comprises the antibody defined in any one of the embodiments according to the first aspect of the invention and/or the functional nucleic acid defined in any one of the embodiments according to the second aspect of the invention, and a pharmaceutically acceptable carrier, and wherein the cancer is selected from LUAD, OV, THCA, and UCEC.

As defined herein, pharmaceutically acceptable carriers suitable for use in the invention are well known by the skilled in the art. Such carriers include, without limitation, water, saline, buffered saline, phosphate buffer, alcoholic/aqueous solutions, emulsions or suspensions. Other conventionally employed diluents, adjuvants and excipients, may be added in accordance with conventional techniques. Such carriers can include ethanol, polyols, and suitable mixtures thereof, vegetable oils, and injectable organic esters. Buffers and pH adjusting agents may also be employed. Buffers include, without limitation, salts prepared from an organic acid or base. Representative buffers include, without limitation, organic acid salts, such as salts of citric acid, e.g., citrates, ascorbic acid, gluconic acid, histidine-HCI, carbonic acid, tartaric acid, succinic acid, acetic acid, or phthalic acid, Tris, tromethamine hydrochloride, or phosphate buffers. Parenteral carriers can include sodium chloride solution, Ringer's dextrose, dextrose, trehalose, sucrose, and sodium chloride, lactated Ringer's or fixed oils. Intravenous carriers can include fluid and nutrient replenishers, electrolyte replenishers, such as those based on Ringer's dextrose and the like. Preservatives and other additives such as, for example, antimicrobials, antioxidants, chelating agents (e.g., EDTA), inert gases and the like may also be provided in the pharmaceutical carriers. The present invention is not limited by the selection of the carrier. The preparation of these pharmaceutically acceptable compositions, from the above-described components, having appropriate pH isotonicity, stability and other conventional characteristics is within the skill of the art. See, e.g., texts such as Remington: The Science and Practice of Pharmacy, 20th ed, Lippincott Williams & Wilkins, publ., 2000; and The Handbook of Pharmaceutical Excipients, 4.sup.th edit., eds. R. C. Rowe et al, APhA Publications, 2003.

The third aspect of the invention also refers to a method of treating cancer in a subject in need thereof, comprising administering to the subject a therapeutically effective amount of a composition as defined above in any of the embodiments of the third aspect of the invention, wherein the cancer is selected from LUAD, OV, THCA, and UCEC. The third aspect of the invention also refers to the use of a composition as defined above in any of the embodiments of the third aspect of the invention for the preparation of a medicament for the treatment cancer, wherein the cancer is selected from LUAD, OV, THCA, and UCEC.

In a particular embodiment of the third aspect according to the previous two paragraphs LUAD is LUAD KRAS G12X, more particularly LUAD KRAS G12C, LUAD KRAS G12V, LUAD KRAS G12D or LUAD KRAS G12A. Preferably, the cancer is LUAD KRAS G12C.

As explained above and shown in Example 1, the inventors have shown that TMPRSS6, CLDN10 or a combination of both serve as prognosis markers and a higher expression of any of them is indicative of a poor prognosis in LUAD. Thus, a **fourth aspect** of the present invention refers to a prognostic method for the prognosis of LUAD comprising the steps of:
a. quantifying the expression level of TMPRSS6 and/or CLDN10, in a sample isolated from an individual who has LUAD (test sample);
b. comparing the levels obtained in step (a) with a reference value or with the expression levels of TMPRSS6 and/or CLDN10 in a reference sample; and
c. assigning the individuals having an expression level of TMPRSS6 and/or CLDN10 obtained in step (b) that is greater than the reference value or the expression level of the reference sample to the group of individuals with a poor prognosis.

In a particular embodiment of the fifth aspect of the invention, the method is an *in vitro* prognostic method.

In a particular embodiment of the fourth aspect of the invention, LUAD is LUAD KRAS G12X, more particularly LUAD KRAS G12C, LUAD KRAS G12V, LUAD KRAS G12D or LUAD KRAS G12A, and preferably, LUAD KRAS G12C.

In a particular embodiment of the fourth aspect of the invention, the level of expression of CLDN10 is quantified, and LUAD is LUAD KRAS G12X, more particularly LUAD KRAS G12C, LUAD KRAS G12V, LUAD KRAS G12D or LUAD KRAS G12A, and preferably, LUAD KRAS G12C.

In a preferred embodiment of the fourth aspect of the invention, the level of expression of TMPRSS6, orTMPRSS6 and CLDN10, is quantified, and LUAD is LUAD KRAS G12X, and more preferably LUAD KRAS G12C, LUAD KRAS G12V, LUAD KRAS G12D or LUAD KRAS G12A, and even more preferably LUAD KRAS G12C.

Preferably, the level of expression of both TMPRSS6 and CLDN10 is quantified and/or the cancer is LUAD KRAS G12C.

As shown in Example 1, the quantification of both TMPRSS6 and CLDN10 gives a synergistically better prognosis result in terms of both OS and FP.

In a particular embodiment of the fourth aspect of the invention, the poor prognosis is characterized by OS and/or FP, preferably OS.

In a preferred embodiment according to any of the embodiments of the fourth aspect of the invention, the sample isolated from the individual is a tissue sample, a bronchial aspirate (BAS), a bronchioalveolar lavage (BAL), or blood sample, and/or the quantification of the expression levels is carried out by means of mRNA or protein quantification.

In the present invention, the level of a mRNA can be determined by methods well known in the art. For example, the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labelled cDNA) and/or amplification (e.g., RT-PCR). Other methods of amplification include ligase chain reaction (LCR), transcription-mediated amplification (TMA), strand displacement amplification (SDA) and nucleic acid sequence-based amplification (NASBA). Conventional methods for quantifying mRNA expression levels can be found, for example, in Sambrook et al., "Molecular cloning: A Laboratory Manual", fourth edition (2012) Cold 35 Spring Harbor Laboratory Press, N.Y., Vol. 1-3. Preferably, the quantity of mRNA is measured by quantitative or semi-quantitative RT-PCR or by real-time quantitative or semi-quantitative RT-PCR.

The level of a protein can be determined in the present invention by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. There is a wide variety of known tests that can be used according to the present invention, such as combined application of non-labelled antibodies (primary antibodies) and labelled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligandbinding assays. Preferred methods to determine the quantity of a protein, or the protein level in a sample, are liquid chromatography followed by mass-spectrometry, or western blot.

Finally, as indicated above, the inventors have seen that TMPRSS6 and CLDN10 have an increased expression level in various cancer types (Figure 4), and as these proteins are surface proteins, they can serve a target-molecules for the delivery of different therapeutic agents. Said therapeutic agents have affinity and/or are able to bind to said target molecules, directly or indirectly, advantageously allowing a specific delivery to the cancer cells. Thus, in a **fifth aspect,** the present invention refers to TMPRSS6 and/or CLDN10 for use in the treatment of cancer as target molecule(s) for delivery of a therapeutic agent or therapy, wherein the cancer is selected from LUAD, OV, THCA and UCEC.

The fifth aspect of the invention also refers to the use of TMPRSS6 and/or CLDN10, preferably TMPRSS6 and CLDN10, as target molecule(s) for delivery of a therapeutic agent or therapy to a cancer cell, wherein the cancer is selected from LUAD, OV, THCA and UCEC.

The fifth aspect of the invention also refers to a method for delivering a therapeutic agent or therapy to a cancer cell wherein TMPRSS6 and/or CLDN10, preferably TMPRSS6 and CLDN10, are used or provided as target molecule(s) and the cancer is selected from LUAD, OV, THCA and UCEC.

In a particular embodiment of the fifth aspect of the invention, the cancer is LUAD, more particularly is LUAD KRAS G12X, even more particularly LUAD KRAS G12C, LUAD KRAS G12V, LUAD KRAS G12D or LUAD KRAS G12A, and preferably LUAD KRAS G12C.

In another preferred embodiment of the fifth aspect of the invention, the therapeutic agent or therapy is selected from the group consisting of immunotherapy, chemotherapy, targeted therapy and/or radioimmunotherapy.

The particular and preferred embodiments of the antibody, functional nucleic acid and/or cancer described for the first aspect of the invention are applicable to the rest of the aspects of the invention.

### REFERENCES

Altschul S.F. et al. Basic local alignment search tool. J Mol Biol. 1990 Oct 5; 215(3):403-101.

Goldman MJ, Craft B, Hastie M, Repečka K, McDade F, Kamath A, et al. Visualizing and interpreting cancer genomics data via the Xena platform. Nature Biotechnology [Internet]. 2020;38(6):675-8.

Györffy B. Survival Analysis Across the Entire Transcriptome Identifies Biomarkers With the Highest Prognostic Power in Breast Cancer. Comput Struct Biotechnol J (2021) 19:4101-9. doi: 10.1016/j.csbj.2021.07.014 s.

Li T, Fu J, Zeng Z, Cohen D, Li J, Chen Q, et al. TIMER2.0 for analysis of tumor-infiltrating immune cells. Nucleic acids research. 2020 Jul;48(W1): W509-14.

Settleman J, Neto JMF, Bernards R. Thinking Differently about Cancer Treatment Regimens. Cancer discovery. 2021 May;11(5):1016-23.

Xiang Z, Zhong C, Chang A, Ling J, Zhao H, Zhou W, et al. Immune-related key gene CLDN10 correlates with lymph node metastasis but predicts favorable prognosis in papillary thyroid carcinoma. Aging. 2020 Feb;12(3):2825-39.

Yang W, Li L, Zhang K, Ma K, Gong Y, Zhou J, et al. CLDN10 associated with immune infiltration is a novel prognostic biomarker for clear cell renal cell carcinoma. Epigenomics. 2021 Jan;13(1):31-45.

Zeng M, Xiong Y, Safaee N, Nowak RP, Donovan KA, Yuan CJ, et al. Exploring Targeted Degradation Strategy for Oncogenic KRAS(G12C). Cell chemical biology. 2020 Jan;27(1): 19-31.e6.

Zhou Y, Xiang J, Bhandari A, Guan Y, Xia E, Zhou X, et al. CLDN10 is Associated with Papillary Thyroid Cancer Progression. Journal of Cancer. 2018;9(24):4712-7.

### EXAMPLES

Specific embodiments of the invention that serve to illustrate the invention without limiting the scope thereof are described in detail below.

### EXAMPLE 1.- Lung cancer prognosis

The KM Plotter Online tool (Győrffy 2021) (https://kmplot.com/analysis/, last accessed on January 2022) was used to evaluate the relationship between up-regulated gene's expression and clinical outcome in patients with LUAD. This open access database contains 3,452 lung cancer samples and allowed us to investigate FP and OS of upregulated genes in the Lung Adenocarcinoma subtype. False discovery rate (FDR) indicates replicable associations across multiple studies.

As shown in Figure 1, the analysis of CLDN10 demonstrated a clear association with worse prognosis, FP (HR:1.56, 95% Cl 1.13-2.16, log rank p = 0.0069); and OS (HR:1.7, 95% Cl 1.3-2.23, log rank p = 7.9 x 10-5) (Figure 1A). A similar finding was observed with TMPRSS6, for FP (HR:1.56, 95% CI 1.13-2.16, log rank p = 0.0069) and OS, (HR:1.49, 95% CI 1.18-1.88, log rank p = 0.00073) (Figure 1B). The combined evaluation of both genes (CLDN10 and TMPRSS6) demonstrated a stronger prediction, for FP: (HR:1.77, 95% Cl 1.27-2.46, log rank p = 6 x 10-4), and OS: (HR:1.75, 95% Cl 1.33-2.29, log ran k p = 4.2 x 10-5) (Figure 1C). Globally, these data demonstrate the association of these two genes with detrimental prognosis in LUAD.

### EXAMPLE 2.- Expression in LUAD

The expression of *TMPRSS6* and *CLDN10* genes was analysed in LUAD samples with and without the KRAS G12C mutation.

The analysis comparing the expression level was done using data from UCSC Xena portal (http://xena.ucsc.edu/, accessed on October 2021) (Goldman et al., 2020) for TCGA LUAD tumour samples.

As shown in Figure 2, the data obtained show a higher expression of *CLDN10* (Figure 2A) and *TMPRSS6* (Figure 2B) when K-RAS harbors the mutation G12C in LUAD tumour samples (TCGA data).

Thus, by interrogating TCGA LUAD samples with KRAS G12C mutated tumours, 2 genes that were highly expressed compared with normal tissues were identified. Interestingly, an increase in the expression level of these same genes was, as shown in Example 1, associated with a poor prognosis in LUAD patients.

### EXAMPLE 3.- Association with immune population

To explore the associations between gene expression and immune infiltration cells TIMER2.0 (http://timer.cistrome.org/, accessed on July 2021) was used. TIMER provides 4 modules (Gene, Mutation, sCNA and Outcome) to explore the association between immune infiltrates and genomic changes (Li et al., 2020). Gene-correlation module was used to link gene expression with activation of T cell markers.

As shown in Figure 3, the expression of neither *CLDN10* gene nor *TMPRSS6* gene were associated with any immune population. Interestingly, this suggests that CLDN10 and TMPRSS6 are present in the primary tumour and not in the stromal compartment, allowing a specific delivery of the therapy targeted to CLDN10 and/or TMPRSS6 to the core of the tumour.

### EXAMPLE 4.- Expression in different tumours

The expression level of *TMPRSS6* gene or *CLDN10* gene individually and their combination was analysed in multiple tumours.

The analysis comparing the expression level of individual genes and combined signature between normal tissue samples and tumour tissue samples was performed with GTEx and TCGA data using GEPIA2 web server (Gene Expression Profiling Interactive Analysis; http://gepia2.cancerpku.cn/) (last accessed on January 2022).

A higher expression of *TMPRSS6* gene or *CLDN10* gene individually could be detected in other tumours (data not shown), and a higher combined expression was observed in LUAD, OV, THCA and UCEC, being the difference in expression statistically significant (*) (Figure 4A).

Moreover, it was seen that the expression of both genes was higher in both LUAD LUSC samples compared with normal tissue samples, however, the increase in expression was only statistically significant (*) in LUAD, no in LUSC, what aligns with the high frequency of K-RAS G12C mutation in LUAD (Figure 4B

## Claims

1. An antibody for use in the treatment of cancer, wherein the antibody is an anti-TMPRSS6 and/or an anti-CLDN10, and the cancer is selected from lung adenocarcinoma, ovarian serous cystadenocarcinoma, thyroid carcinoma and uterine corpus endometrial carcinoma.

2. The antibody for use according to claim 1, wherein the antibody is a combination of an anti-TMPRSS6 antibody and an anti-CLDN10 antibody.

3. The antibody for use according to claim 1 or 2, wherein the cancer is lung adenocarcinoma.

4. The antibody for use according to any one of claims 1 to 3, wherein the cancer is lung adenocarcinoma KRAS G12C.

5. The antibody for use according to any one of claims 1 to 4, wherein the antibody is a functional fragment of the antibody, preferably said fragment is selected from the group comprising or consisting of Fab, Fab', F(ab')2, Facb, scFv, diabodies, monobodies, and nanobodies.

6. The antibody for use according to any one of claims 1 to 5, wherein the antibody is a monoclonal antibody, a chimeric antibody, a human antibody or a humanized antibody, preferably it is a monoclonal humanized antibody.

7. The antibody for use according to any one of claims 1 to 6, wherein the antibody is a bi-specific antibody, a T-cell engager, an antibody drug conjugate, or a chimeric antigen receptor.

8. Functional nucleic acid for use in the treatment of cancer, **characterized in that** a transcript of TMPRSS6 and/or CLDN10 is the target of said functional nucleic acid, and the cancer is selected from lung adenocarcinoma, ovarian serous cystadenocarcinoma, thyroid carcinoma and uterine corpus endometrial carcinoma, preferably the functional nucleic acid is selected from siRNA, shRNA or miRNA, and/or the cancer is lung adenocarcinoma KRAS G12C.

9. A pharmaceutical composition for use in the treatment of cancer, wherein the pharmaceutical composition comprises the antibody defined in any one of claims 1 to 7 or the functional nucleic acid defined in claim 8, and a pharmaceutically acceptable carried, and the cancer is selected from lung adenocarcinoma, ovarian serous cystadenocarcinoma, thyroid carcinoma and uterine corpus endometrial carcinoma, preferably the cancer is lung adenocarcinoma KRAS G12C.

10. The pharmaceutical composition for the use according claim 9, further comprising an additional therapeutic agent.

11. A prognostic method for the prognosis of lung adenocarcinoma comprising the steps of:
a. quantifying the expression levels of TMPRSS6, or TMPRSS6 and CLDN10, in a sample isolated from an individual who has lung adenocarcinoma;
b. comparing the levels obtained in step (a) with a reference value or with the expression levels of TMPRSS6, or TMPRSS6 and CLDN10, in a reference sample; and
c. assigning the individuals having expression levels of TMPRSS6, or TMPRSS6 and CLDN10, obtained in step (b) that are greater than the reference value or the expression levels of the reference sample to the group of individuals with a poor prognosis.

12. A prognostic method for the prognosis of lung adenocarcinoma KRAS G12X comprising the steps of:
a. quantifying the expression levels of TMPRSS6 and/or CLDN10 in a sample isolated from an individual who has lung adenocarcinoma KRAS G12X;
b. comparing the levels obtained in step (a) with a reference value or with the expression levels of TMPRSS6 and/or CLDN10 in a reference sample; and
c. assigning the individuals having expression levels of TMPRSS6 and/or CLDN10 obtained in step (b) that are greater than the reference value or the expression levels of the reference sample to the group of individuals with a poor prognosis, wherein X is selected from the group consisting of C, V, D or A, preferably X is C

13. The method according to claim 11 or 12, wherein the expression level of both TMPRSS56 and CLDN10 is quantified.

14. The method according to any one of claims 11 to 13, wherein the sample isolated from the individual is a tissue sample, a bronchial aspirate (BAS), a bronchioalveolar lavage (BAL), or blood sample and/or the quantification of the expression levels is performed by means of mRNA or protein quantification.

15. TMPRSS6 and/or CLDN10 for use in the treatment of cancer as target molecule(s) for delivery of a therapy, wherein the cancer is selected from lung adenocarcinoma, ovarian serous cystadenocarcinoma, thyroid carcinoma and uterine corpus endometrial carcinoma.
